(19) Europäisches Patentamt / European Patent Office / Office européen des brevets

(11) **EP 4 220 646 A1**

(12) **EUROPEAN PATENT APPLICATION**
published in accordance with Art. 153(4) EPC

(43) Date of publication:
**02.08.2023 Bulletin 2023/31**

(21) Application number: **21871710.6**

(22) Date of filing: **17.11.2021**

(51) International Patent Classification (IPC):
$G16B\ 50/00^{(2019.01)}$     $G16C\ 20/70^{(2019.01)}$

(52) Cooperative Patent Classification (CPC):
**G16B 50/00; G16C 20/70**

(86) International application number:
**PCT/CN2021/131105**

(87) International publication number:
**WO 2022/063341 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.09.2020   CN 202011033081
26.11.2020   CN 202011343180**

(71) Applicant: Shenzen Taili Biotechnology Co., Ltd.
**Shenzhen, Guangdong 518548 (CN)**

(72) Inventors:
• **CHEN, Liang
Shenzhen, Guangdong 518548 (CN)**

• **KASIM, Mamtimin
Shenzhen, Guangdong 518548 (CN)**
• **ZHANG, Xiangtao
Shenzhen, Guangdong 518548 (CN)**
• **LAN, Wanjun
Shenzhen, Guangdong 518548 (CN)**
• **LEUNG, Kingsley
Shenzhen, Guangdong 518548 (CN)**
• **LIANG, Chuheng
Shenzhen, Guangdong 518548 (CN)**

(74) Representative: **Hoffmann Eitle
Patent- und Rechtsanwälte PartmbB
Arabellastraße 30
81925 München (DE)**

(54) **BASAL CULTURE MEDIUM DEVELOPMENT METHOD, BASAL CULTURE MEDIUM FORMULATION AND DEVELOPMENT, AND SYSTEM THEREOF**

(57)     The present application provides a method for developing a basal culture medium, a method for developing a basal culture medium formulation and system thereof. The method for developing a basal culture medium comprises, (1) determining a regression model for selected culture indicators to predict the culture indicators of a basal culture medium; (2) acquiring an addition range of each component in the basal culture medium, and enumerating and randomly selecting to generate a plurality of candidate basal culture medium formulations; (3) predicting the culture indicators by adopting the regression model and recommending a basal culture medium formulation; and (4) performing cell culture experiments to verify the culture indicators of the recommended basal culture medium formulation.

FIG. 1

**Description**

CROSS-REFERENCE TO RELATED APPLICATIONS

The present application claims priority to Chinese patent application No. 2020113431805, filed on November 26, 2020, and titled "Basal culture medium development method based on culture indicator evaluation"; and Chinese patent application No. 2020110330817, filed on September 27, 2020, and titled "Basal culture medium formulation development method and system based on artificial intelligence", the contents of which are hereby incorporated by reference in their entirety.

TECHNICAL FIELD

The present application belongs to the field of biotechnology, and more particularly, relates to a method for developing a basal culture medium, and a method for developing a basal culture medium formulation and a system thereof.

BACKGROUND

Serum-free, animal-derived component-free, chemically-defined basal culture medium consists of carbon sources, amino acids, vitamins, trace metal ions, lipids, buffered reagents, and other additive reagents. The traditional method for developing a basal culture medium formulation is based on one or several classical medium(s), such as DEME/F12. A single factor test or a DOE screening test is used to find out the key components by adding a variety of different components, and then many DOEs designs of experiments such as a response surface method or the like can be adopted to optimize the concentration of each component to obtain an optimal formulation. Alternatively, a formulation can be optimized based on cell metabolism analysis, genomics analysis and proteomics analysis to identify the changes of each component during the process of cell growth and their effects on yield and quality of a target product.

The existing technology requires multiple rounds of tests, and each test could not cover all components, which takes a long time and requires more professional theoretical knowledge of basic chemistry, biochemistry, molecular biology, cell biology, etc. Moreover, the resultant formulation may not be the optimal one.

The traditional method for developing a basal culture medium formulation is based on one or several classical medium, such as DEME/F12. A single factor test or a DOE screening test is used to find out the key components by adding a variety of different components, and then many DOEs designs of experiments such as a response surface method or the like can be adopted to optimize the concentration of each component to obtain an optimal formulation. Alternatively, a formulation can be optimized based on cell metabolism analysis, genomics analysis and proteomics analysis to identify the changes of each component during the process of cell growth and their effects on yield and quality of a target product.

SUMMARY

In view of the above defects or requirements for improvement of the prior art, the present application provides a method for developing a basal culture medium, and a method for developing a basal culture medium formulation and a system thereof.

In one aspect, the present application provides a method for developing a basal culture medium based on culture indicators evaluation, comprising the following steps of:

    (1) Selecting and optimizing a regression model for selected culture indicators through an experimentally verified basal culture medium formulation database, to determine a regression prediction model for predicting the culture indicators of a basal culture medium;
    (2) Acquiring an addition range of each component in the basal culture medium, and enumerating and randomly selecting addition amount of each component, to obtain a plurality of candidate basal culture medium formulations;
    (3) Predicting the culture indicators of the candidate basal culture medium formulations obtained in step (2) by adopting the regression prediction model obtained in step (1), and screening one or more formulation(s) from the candidate basal culture medium formulations as recommended basal culture medium formulations based on prediction results; and
    (4) Conducting cell culture experiments with the recommended basal culture medium formulations obtained in step (3), to verify the culture indicators of the recommended basal culture medium formulations and determine an optimal basal culture medium formulation accordingly.

Preferably, in the method for developing a basal culture medium based on culture indicators evaluation, the step (1) of determining a regression prediction model for predicting the culture indicators of a basal culture medium, comprises the following steps of:

(1-1) Collecting training formulations

A training formulation is formed by searching within the addition range of each component, which includes, but is not limited to, the following three methods, i.e., random generation of formulation, DOE design of experiment formulation, or formulation formation by mixing.

(1-2) Acquiring culture indicator data

Culture indicator data of the training formulation obtained in step (1-1) is acquired by conducting cell culture experiments,

(1-3) Creating a training sample data set

A training sample data set is created with the addition amount of each component in the training formulation or its normalized value obtained in step (1-1) as an input matrix and the culture indicator data obtained in step (1-2) as an output matrix; and

(1-4) training and testing the multiple alternative regression prediction models with the training sample data set obtained in step (1-3) under the same conditions, and selecting the optimal one as the regression prediction model for predicting the culture indicators of a basal culture medium.

The present application provides a method for developing basal culture medium formulation and a system thereof based on artificial intelligence. The object of the present application is to apply machine learning algorithms to complex processes of formulation optimization. The most potential basal culture medium formulation with good culture effects can be recommended in a short time by creating a high-quality and abundant sample formulation database, and selecting appropriate machine learning algorithms and optimization algorithms. Therefore, it would be easier to develop formulation, and thus could solve the technical problems of slow development rate and high development cost due to the complex components of the existing basal culture medium.

In one aspect, the present application provides a method for developing a basal culture medium formulation based on artificial intelligence, comprising the following steps of:

(1) Creating a sample formulation database

Components in candidate basal culture medium formulations are acquired, a search space of addition proportion for each component is determined, basal culture medium sample formulations are formed by searching within the search space for each component, and a sample formulation database is created by collecting the basal culture medium sample formulations.

(2) Obtaining a sample formulation culture database

Culture effects of each basal culture medium sample formulation stored in the sample formulation database obtained in step (1) are acquired by conducting experimental verification based on development purpose, and basal culture medium sample formulation data associated with culture effects are collected as a sample formulation culture database.

(3) Training a machine learning model by the sample formulation culture database obtained in step (2) based on development purpose, to obtain a model for predicting culture effects of a basal culture medium formulation.

(4) Predicting culture effects by regression adopting the model for predicting culture effects of a basal culture medium formulation obtained in step (3) within the search space of addition proportion for each component in the basal culture medium formulation to be optimized based on development purpose, and recommending an optimal basal culture medium formulation based on the predicted culture effects.

In yet another aspect, the present application provides a system for developing a basal culture medium formulation based on artificial intelligence, comprising a sample formulation generation module, a sample formulation culture database, a regression model training module, and a formulation recommendation module.

The sample formulation generation module is configured for forming basal culture medium sample formulations by searching within the search space of addition proportion for each component in the basal culture medium formulation to be optimized, and creating a sample formulation database.

The sample formulation culture database can store each basal culture medium sample formulation in the sample formulation database and the associated culture effects data.

The regression model training module is configured for selecting and training a regression model by using the basal culture medium sample formulations and the associated culture effects data stored in the sample formulation culture database, to obtain and store a model for predicting culture effects of a basal culture medium formulation.

The formulation recommendation module is configured for applying the model for predicting culture effects of a basal culture medium formulation stored in the regression model training module to predict culture effects of a basal culture medium formulation within the search space, and recommending an optimal basal culture medium formulation.

In general, comparing with the prior art, the above technical solutions conceived in the present application has beneficial effects.

BRIEF DESCRIPTION OF THE DRAWINGS

FIG. 1 is a schematic flow chart of the method for developing a basal culture medium based on culture indicator evaluation according to the present application.

FIG. 2 is a schematic flow chart of the method for developing basal culture medium based on culture indicator evaluation according to an embodiment of the present application.

FIG. 3 is cell density curves for batch cultures of the optimal 3 basal culture medium formulations obtained in an embodiment of the present application.

FIG. 4 is a schematic flow chart of the method for developing basal culture medium based on artificial intelligence according to the present application.

FIG. 5 is a prediction accuracy evaluation of various machine learning models for maximum cell density in the sample formulation database within 7 days using 15-fold cross-validation.

FIG. 6 is a prediction accuracy evaluation of various machine learning models for cell density in the sample formulation database on day 5 using 15-fold cross-validation.

FIG. 7 is a schematic flow chart of the method for developing a basal culture medium formulation according to an embodiment of the present application.

FIG. 8 is a comparison of the predicted value with the actual value data of the basal culture medium formulation recommended for maximum cell density within 7 days according to an embodiment of the present application.

FIG. 9 is a cell growth curve of the basal culture medium formulation recommended for maximum cell density within 7 days according to an embodiment of the present application.

FIG. 10 is a comparison of the predicted value with the actual value data of the basal culture medium formulation recommended for cell density on day 5 according to an embodiment of the present application.

FIG. 11 is a cell growth curve of the basal culture medium formulation recommended for cell density on day 5 in accordance with an embodiment of the present application.

DETAILED DESCRIPTION

The present application will now be described in detail with reference to the accompanying drawings and embodiments in order to clarify the objects, technical solutions, and advantages of the present application. It should be understood that the specific embodiments described herein are only for explaining the present application, and not intended to limit the present application. Moreover, the technical features involved in the various embodiments of the present application described below may be combined provided that they do not conflict with each other.

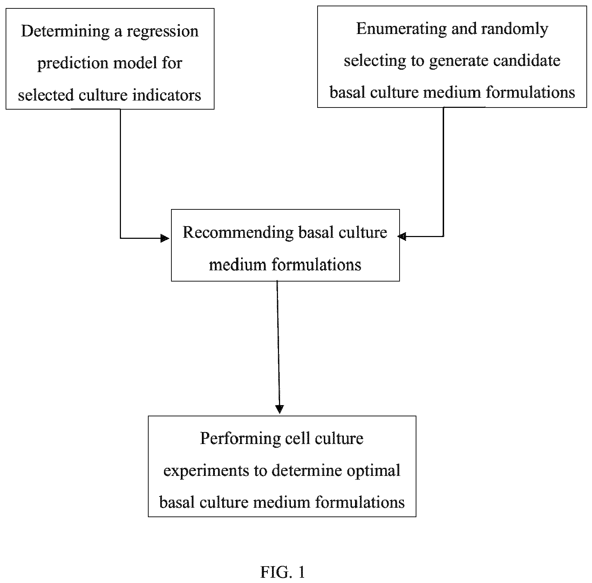

The present application provides a method for developing a basal culture medium based on culture indicator evaluation, as shown in FIG. 1, comprising the following steps of:

(1) Selecting and optimizing a regression model for selected culture indicators through an experimentally verified basal culture medium formulation database, to determine a regression prediction model for predicting the culture indicators of a basal culture medium.

The step of determining a regression prediction model for predicting the culture indicators of a basal culture medium comprises the following steps of:

(1-1) Collecting a training formulation

A training formulation is formed by searching within the addition range of each component, which includes, but is not limited to, the following three methods, i.e., a random generation of formulation, DOE design of experiment formulation or formulation formation by mixing.

The random generation of formulation means that a base cultural medium sample formulation is formed by randomly taking value within the addition range of each component in the basal culture medium formulation.

The DOE design of experiment formulation comprises the following steps of:

S1-clustering the lowest addition proportion of each component in the basal culture medium to obtain multiple addition magnitudes, and dividing each component in the basal culture medium into functional categories based on its functions, wherein the functional categories include amino acids, trace metal ions, vitamins, lipids, buffered reagents and the like; and

S2-forming a DOE experimental factor by combining the multiple addition magnitudes and functional categories obtained in the step S1, and forming a basal sample formulation by using a space-filling DOE design of experiment, wherein the space-filling DOE experiment design includes a ball filling method, a Latin hypercube method, a uniform method and a minimum potential method, and it is preferably to use the Latin hypercube method to design formulations.

The formulation formation by mixing means that a new basal culture medium sample formulation is obtained by screening and combining existing basal culture medium sample formulations; preferably, the following method is used for screening and combining existing basal culture medium sample formulations, i.e., verifying culture effects of the existing basal culture medium sample formulations, selecting formulations with a high cell survival rate, a high cell density or a high protein expression level, and forming a new formulation by mixing the above two or three or more formulations in random or preset proportions.

(1-2) Acquiring culture indicator data

Culture indicator data of the training formulation obtained in step (1-1) is acquired by conducting cell culture experiments.

(1-3) Creating a training sample data set

A training sample data set is created with the addition amount of each component in the training formulation or its normalized value obtained in step (1-1) as an input matrix and the culture indicator obtained in step (1-2) as an output matrix.

In order to ensure the universality of the data, the acquired culture indicators of the training formulations should include as many culture indicators as possible, including but not limited to a cell survival rate, a cell density, a protein expression level, glucose, lactic acid, or ammonia;

Specifically, a set of experimental data consists of input data $(x_1, x_2, ..., x_n)$ and output data $(y_1, y_2, ..., y_m)$; wherein $x_i$ is the $i^{th}$ component of the basal culture medium formulation, used as a feature during model training, verification and testing; $y_1$ represents a cell survival rate, $y_2$ represents a cell density, $y_3$ represents a protein expression level, ..., $y_m$ represents the $m^{th}$ output indicator, including but not limited to, glucose, lactic acid, ammonia or the like. The input matrix of the regression model is shown as X matrix, wherein $x_{ij}$ represents the $j^{th}$ component of the $i^{th}$ formulation; and the output matrix is shown as Y matrix, wherein $y_{ij}$ represents the $j^{th}$ output value of the $i^{th}$ formulation.

$$X = \begin{pmatrix} x_{11} & x_{12} & \cdots & x_{1n} \\ \vdots & \vdots & \cdots & \vdots \\ x_{n1} & x_{n2} & \cdots & x_{nn} \end{pmatrix}$$

$$Y = \begin{pmatrix} y_{11} & y_{12} & \cdots & y_{1m} \\ \vdots & \vdots & \cdots & \vdots \\ y_{n1} & y_{n2} & \cdots & y_{nm} \end{pmatrix}$$

Preferably, the components in the formulation can be optimized through feature selection of the model.

(1-4) Training and testing the multiple alternative regression prediction models with the training sample data set obtained in step (1-3) under the same conditions, and selecting the optimal one as the regression prediction model for predicting the culture indicators of a basal culture medium.

The multiple alternative regression prediction models include, but is not limited to, a support vector regression model, an elastic network (ElasticNet) model, a Xgboost model, a Gradient Boosting Regression model, a Logostic Regression model, a regression model based on multi-layer neural network, a regression model based on convolution neural network, and/or a regression model based on recurrent neural network. Since different regression models have different implementation forms, that is, the underlying mathematical principles are different, different regression models can be adopted to predict the output of a basal culture medium. Since different regression models have different implementation forms, that is, the underlying mathematical principles are different, the output prediction performance of a basal culture medium would be different with different regression models. A step of selecting a regression prediction model is required in order to optimize and select. Different regression models are adopted to predict the output of a basal culture medium, and different prediction results may be obtained by different regression prediction models for different culture indicators. Therefore, the regression prediction model with best performances is selected by comparing the performances of the different regression models.

The step of training and testing with the training sample data set under the same conditions preferably includes dividing the training sample data set into training, verification, and testing by a ratio of 8:1:1 to conduct crossover experiments, wherein verification is used to avoid over-fitting of the data, and testing is used to evaluate model generalization ability and prediction ability. A regression prediction model can be selected for different prediction using MSE mean-square error, root mean square error RMSE, and/or R-SQUARED ability based on prediction purpose.

(2) Acquiring an addition range of each component in a basal culture medium, and enumerating and randomly selecting addition amount of each component, to obtain a plurality of candidate basal culture medium formulations. Specifically, the addition amount of each component is enumerated as many and uniform values as possible within the addition range of each component, thereby forming a large formulation data.

According to the present application, means such as avoiding model overfitting, enumerating addition amount of component or the like are combined, so that an optimization trap in which a local optimal solution is used as a global optimal solution due to experimental experiences is avoided, and a global optimal basal culture medium formulation is obtained efficiently.

Specifically, the following steps are included.

(2-1) Acquiring a point value with the same number within a value range of each component in the basal culture medium, and forming a value sequence for each component;
Preferably, the point value with the same number constitutes an arithmetic sequence or a geometric sequence, which has a better uniformity and can better cover a value range thereof.
(2-2) Disorderly sorting the value sequence of all components obtained in step (2-1) to obtain a rearranged component value sequence;
(2-3) Constructing a component value matrix by taking the rearranged component value sequence obtained in step (2-2) as a line or a column, and obtaining a candidate basal culture medium formulation by taking the column or the line of the component value matrix as a value of each component in a formulation.
(2-4) Repeating the above steps (2-1) to (2-3) to obtain more candidate basal culture medium formulations. Preferably, the number of the candidate basal culture medium formulations is 1,000-1,000,000.
(3) Predicting culture indicators of the candidate basal culture medium formulations obtained in step (2) by the regression prediction model obtained in step (1), and screening one or more recommended basal culture medium formulation(s) from the candidate basal culture medium formulations based on prediction results; and
(4) Conducting cell culture experiments with the recommended basal culture medium formulations obtained in step (3), to verify the culture indicators of the recommended basal culture medium formulations and determine an optimal basal culture medium formulation accordingly.

Regression analysis is a predictive modeling technique that studies the relationship between dependent variables (targets) and independent variables (predictors). By means of regression analysis, a model between each component of the culture medium (independent variables) and a protein expression level, a cell density, or a cell survival rate, etc. (dependent variable) is first built by using experimental data of the existing basal culture medium formulations, and then a large number of output indicators of the basal culture medium formulation are predicted by adopting the model, that is, indicators that will be generated after the basal culture medium formulation is used, such as a protein expression level, a survival rate, a density, or the like, are predicted. A large formulation data may be formed according to the value range of each component in the formulation. The value range of the artificially optimized formulation data is narrow, and the value range of each component in the automatically formed formulation data is comprehensive. A basal culture medium formulation that conforms to one or more indicator(s) is then selected from a number of prediction results as a candidate basal culture medium formulation. Cells were finally cultured in a candidate basal culture medium to verify the basal culture medium formulation. A candidate basal culture medium formulation that meets the screening requirements in a verified experiment is identified as an optimal basal culture medium formulation, thereby greatly reducing the number of experiments, saving labors and financial resources, and shortening the development time.

The present application provides a system for developing a basal culture medium based on culture indicator evaluation, comprising a regression model selection module, a candidate basal culture medium formulation generation module, and a basal culture medium formulation recommendation module.

The regression model selection module is configured for selecting and optimizing a regression model through an experimentally verified basal culture medium formulation database, to determine a regression prediction model for predicting the culture indicators of a basal culture medium and submit it to the basal culture medium formulation recommendation module.

The candidate basal culture medium formulation generation module is configured for acquiring an addition range of each component in a basal culture medium, and enumerating and randomly selecting addition amount of each component, to obtain a plurality of candidate basal culture medium formulations and submit them to the basal culture medium formulation recommendation module. The candidate basal culture medium formulation generation module includes an enumeration sub-module, a rearrangement sub-module, and a combination sub-module.

The enumeration sub-module is configured for acquiring a point value with the same number within a value range of each component in a basal culture medium, to form a value sequence of each component, and to submit it to the rearrangement sub-module.

The rearrangement sub-module is configured for disorderly sorting the value sequence of the components, to obtain a rearranged component value sequence, and to submit it to the combination sub-module.

The combination sub-module is configured for constructing a component value matrix by taking the rearranged component value sequence as a line or a column, and obtaining a candidate basal culture medium formulation by taking the column or the line of the component value matrix as a value of each component of the formulation.

The basal culture medium formulation recommendation module is configured for predicting culture indicators of the candidate basal culture medium formulation by adopting the regression prediction model, and screening one or more recommended basal culture medium formulation(s) from the candidate basal culture medium formulations based on prediction results.

The following method is shown as an example.

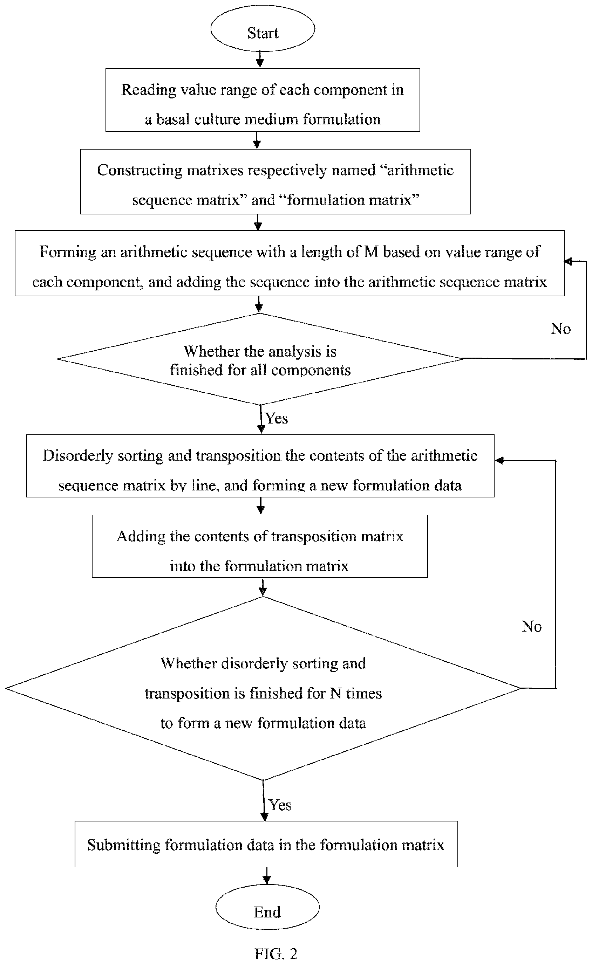

A method for developing a basal culture medium based on culture indicator evaluation, as shown in FIG.2, comprises the following steps of

(1) Selecting and optimizing a regression model for selected culture indicators through an experimentally verified basal culture medium formulation database, to determine a regression prediction model for predicting culture indicators of a basal culture medium.

The step of determining the regression prediction model for predicting culture indicators of a basal culture medium comprises the following steps of:

(1-1) Collecting a training formulation

In this example, a training formulation set is formed by aggregating experimental data from cell culture in randomly formed formulations, DOE experimental design formulations, mixed forming formulation formed by mixing two or more existing culture medium formulations in a certain ratio by using computer AI technology.

A training formulation is formed by searching within the addition range of each component, which includes, but not limits to, the following three methods, i.e., random generation of formulation, DOE design of experiment formulation, or formulation formation by mixing.

Specifically, the random generation of formulation comprises finding out components contained in a culture medium formulation and a concentration range of components by referring to the data, and then randomly taking values within the concentration range for each component and taking one random value of each component to form a formulation.

The DOE design of experiment formulation comprises the following steps of:

S1-clustering the lowest addition proportion of each component in a basal culture medium to obtain multiple addition magnitudes, and dividing each component in a basal culture medium into functional categories based on its functions, wherein the functional categories include amino acids, trace metal ions, vitamins, lipids, buffered reagents, and the like.

Specifically, except for a small number of invariant components in the formulation such as glucose, all the other components are divided into five major categories, including amino acids, trace metal ions, vitamins, lipids, buffered reagents, and the like.

S2-forming a DOE experimental factor by combining the multiple addition magnitudes and functional categories obtained in the step S1, and forming a basal sample formulation by using a space-filling DOE design of experiment.

Specifically, in each major category, each component has a maximum addition value of 100%, and a lowest addition percentage obtained from dividing a minimum value by a maximum value in the formulation. Components with the lowest addition percentage close to each other are selected to form a new category, so that nine major categories, i.e., nine factors, are formed based on the above five major categories. Ninety formulations are designed using a space-filling Latin hypercube DOE design of experiment.

The formulation formation by mixing means that a new basal culture medium sample formulation is obtained by screening and combining existing basal culture medium sample formulations; specifically, the step of screening and combining existing basal culture medium sample formulations includes, verifying culture effects of an existing basal culture medium sample formulation, selecting a formulation with a high cell survival rate, a high cell density, or a high protein expression level, and forming a new formulation by mixing the above two or three or more formulations in random or preset proportions.

(1-2) Acquiring culture indicator data

Culture indicator data of the training formulation obtained in step (1-1) is acquired by conducting cell culture experiments.

Specifically, in order to ensure the universality of the data, the obtained culture indicators of the training formulation should cover as many culture indicators as possible, including a cell survival rate, a cell density, a protein expression level, glucose, lactic acid, or ammonia.

Specifically, a large number of training formulations formed in the three methods according to the above description are used for cell batch culture. The culture container is 50 mL mini bioreactor, a culture volume is 10 mL, a theoretical inoculation density is 0.5E+06 cells/mL, and culture time is 7 days. The samples are counted on day 3 and day 5, and the contents of biochemical parameters such as glucose and lactic acid are detected. The batch culture is terminated after the samples are counted and the parameters such as glucose, lactic acid, and protein expression are detected on day 7.

(1-3) Creating a training sample data set

A training sample data set is created with the addition amount of each component or its normalized value in the training formulation obtained in step (1-1) as an input matrix and the culture indicator obtained in step (1-2) as an output matrix.

A set of experimental data consists of input data $(x_1, x_2, ..., x_n)$ and output data $(y_1, y_2, ..., y_m)$, wherein $x$, is the $i^{th}$ component of a basal culture medium formulation, used as a feature during model training, verification and testing; $y_1$ represents a cell survival rate, $y_2$ represents a cell density, $y_3$ represents a protein expression level, ..., and $y_m$ represents the $m^{th}$ output indicator, including but not limited to the parameters such as glucose, lactic acid, ammonia, or the like. The input matrix of the regression model is shown as X matrix, wherein $x_{ij}$ represents the $j^{th}$ component of the $i^{th}$ formulation. The output matrix is shown as Y matrix, wherein $y_{ij}$ represents the $j^{th}$ output value of the $i^{th}$ formulation.

$$X = \begin{pmatrix} x_{11} & x_{12} & \cdots & x_{1n} \\ \vdots & \vdots & \cdots & \vdots \\ x_{n1} & x_{n2} & \cdots & x_{nn} \end{pmatrix}$$

$$Y = \begin{pmatrix} y_{11} & y_{12} & \cdots & y_{1m} \\ \vdots & \vdots & \cdots & \vdots \\ y_{n1} & y_{n2} & \cdots & y_{nm} \end{pmatrix}$$

Experimental data is preprocessed. Since measurement units for each component and each value of the output indicators in a basal culture medium formulation are different, the training and verification efficiency of the model might be affected if they are directly used. Therefore, it is necessary to perform normalization preprocessing on the input and output data.

This example takes $y_2$, the maximum value of cell density during the 7-day batch culture as an example, and the values of different output indicators at different stages can also be modeled, verified, tested and predicted in the specific implementations.

The basal culture medium formulation data is written into an Excel file, each row representing data associated with one basal culture medium. Data are read directly from Excel files when formulations are trained, tested, or predicted by models. For convenience of processing, the data of the Excel file can be written into other files such as a CSV file, a database file, or the like, and a corresponding file format can be formed.

Features are selected. The contribution of each component in a basal culture medium formulation to the output indicator is different. Therefore, it is necessary to determine which component contributes significantly to the prediction of the output indicator in order to improve training efficiency and reduce prediction error. In order to determine which feature contributes significantly to the output value prediction, different kinds of features are scored by a method such as correlation feature selection, mutual information feature selection, or the like, and a feature with a high score is selected to conduct prediction experiments. When the prediction results reach a highest value, the corresponding feature is the one with the strongest prediction ability of the output value. In specific implementations, the experimental data are pre-processed, and then each feature is scored by using methods such as correlation feature selection, mutual information feature selection, or the like. The higher the score of a component is, the greater its contribution to the output indicator prediction is. The components are sorted from high to low by score value to form one feature score sequence.

It is shown in the feature screening results of this example that the following components contribute significantly to the maximum cell density during the 7-day batch culture and are used as feature values of this example: L-glutamic acid, L-alanine, L-tryptophan, manganese sulfate monohydrate, sodium selenite, cobalt chloride hexahydrate, pyridoxal hydrochloride, sodium pyruvate, HEPES (pH buffered reagent), and sodium bicarbonate.

Two or three regression models with better performances are selected, and the components with the highest score value, such as 5, 10, 15, 20, ..., or all of the components, are selected in different proportions from the score sequence, to conduct regression model training, verification and testing experiments. 10-fold crossover experiments are selected during the experiments to evaluate performances of the regression models based on the average value of 10-fold crossover experiments, such as regression model index including MSE, R2, or the like. Other experiments in this example were similar. The performances of each model with different proportions of components data are then compared, and the components with the most significant contribution to the prediction are selected. The component with the most significant contribution to the prediction means that the performance of the model would not change if the component is added to the model, while the performance would decrease if the component is reduced, and the corresponding component sequence is the component set with the best predicted performances.

(1-4) Training and testing the multiple alternative regression prediction models with the training sample data set obtained in step (1-3) under the same conditions, and selecting the optimal one as the regression prediction model for predicting culture indicators of a basal culture medium.

The multiple alternative regression prediction models include, but are not limited to, a support vector regression model (SVR), an elastic network (ElasticNet) model, a Xgboost model, a Gradient Boosting Regression model, a Logostic Regression model, a regression model based on multi-layer neural network, a regression model based on convolution neural network, or a regression model based on recurrent neural network.

Since different regression models have different implementation forms, that is, the underlying mathematical principles are different, the output prediction performances of a basal culture medium would be different with different regression models. The step of selecting a regression prediction model is required in order to optimize and select. Different regression models are adopted to predict the output of a basal culture medium, and different prediction results for different culture indicators will be obtained with different regression prediction models. Therefore, a regression prediction model with best performances is selected by comparing the performances of the different regression models.

The step of training and testing with the training sample data set under the same conditions includes, dividing the training sample data set into training, verification, and testing in a ratio of 8:1:1 to conduct crossover experiments, wherein verification is used to avoid over-fitting of the data, and testing is used to evaluate model generalization ability and prediction ability. A regression prediction model can be selected for different prediction ability using MSE mean-square error, root mean square error RMSE, and/or R-SQUARED, based on prediction purpose.

Prediction of the basal culture medium can be achieved using different regression algorithms, but prediction accuracy would be different. In order to determine which regression algorithm has a good prediction effect on the basal culture medium, different regression algorithms are used for training and verification, and a regression model is built. Formulation prediction experiments are performed on the same test data using different regression models, and the results are compared to determine which regression model has the best prediction efficiency of the basal culture medium.

In this example, MSE mean square error, root mean square error RMSE, and R-SQUARED are used for synthetically screening of indicators, and a regression model based on a convolutional neural network is selected as the model with best effects.

(2) Acquiring an addition range of each component in a basal culture medium, and enumerating and randomly selecting addition amount of each component, to obtain a plurality of candidate basal culture medium formulations. Specifically, the addition amount of each component is enumerated as many and uniform values as possible within the addition range of each component, thereby forming a large formulation data. The following steps are included in the method.

Step 1, defining an empty matrix, and storing the formulation data formed, named PF.

Step 2, forming a sequence with a length of M based on a value range of each component in a basal culture medium formulation. Any type of sequences can be formed depending on the actual situation. Experimental data in this example is formed as an arithmetic sequence. A user can form an arithmetic sequence with a length of m and a tolerance of d. For example, the value range of $x_i$ is ($x_{i\_min}$, $x_{i\_max}$), forming an arithmetic sequence $\{x_{1\_min}, x_{1\_min}+d, x_{1\_min}+i{*}d, ..., x_{i\_max}\}$, wherein $x_{i\_min}$ is the minimum value of the value range and $x_{i\_max}$ is the maximum value of the value range. The value ranges of all the components are processed according to the above method to obtain an arithmetic sequence matrix as follows.

$$\begin{pmatrix} x_{1\_min} & \cdots & x_{1\_max} \\ \vdots & \cdots & \vdots \\ x_{n\_min} & \cdots & x_{n\_max} \end{pmatrix}$$

Step 3, disorderly sorting each row of the arithmetic sequence matrix to obtain new matrix A with n rows and m columns, and obtaining matrix B with m rows and n columns by transposing matrix A, wherein each row of matrix B represents a newly formed formulation, and m sets of formulations are formed. The length of the arithmetic sequence is m.

$$A = \begin{pmatrix} x_{1_{min}+id} & \cdots & x_{1_{min}+jd} \\ \vdots & \cdots & \vdots \\ x_{n_{min}+kd} & \cdots & x_{min+gd} \end{pmatrix} \qquad B = \begin{pmatrix} x_{1_{min}+id} & \cdots & x_{n_{min}+kd} \\ \vdots & \cdots & \vdots \\ x_{1_{min}+jd} & \cdots & x_{n_{min}+gd} \end{pmatrix}$$

Step 4: adding the data of matrix B into formulation matrix PF in row. Each row of formulation matrix PF represents a new formulation.

Step 5: repeating Step 3 for k times, and adding the transposed matrix into formulation matrix PF, so that the number of formulations is continuously increased to obtain k*m formulations.

By the above steps, a large number of candidate basal culture medium formulations can be obtained by adjusting the length m and the cycle number k of the arithmetic sequence.

(3) Predicting culture indicators of the candidate basal culture medium formulations obtained in step (2) by adopting the regression prediction model obtained in step (1), and screening one or more formulation(s) from the candidate basal culture medium formulations based on prediction results as recommended basal culture medium formulations;

Specifically, the following steps are included in the method.

Step 1: predicting the obtained candidate basal culture medium formulations with a regression model.

Step 2: selecting a formulation that satisfies both conditions $\{y_1 \geq y_1\_optimal \cap y_2 \geq y_2\_optimal \cap y_3 ... \geq y_{m\_optimal}\}$ based on prediction results as a candidate formulation, wherein $y_{1\_optimal}$ is the lowest optimal value of $y_1$, $y_{2\_optimal}$ is the lowest optimal value of $y_2$, and $y_{m\_optimal}$ is the lowest optimal value of $y_m$.

For example, when there are five components in a basal culture medium, an arithmetic sequence with a length of M is formed for each component based on the range of each component in a basal culture medium formulation. The value ranges are shown in Table 1 below.

Table 1. Output Range of Components in Basal Culture Medium Formulation

| Component | Min | Max |
|-----------|-----|-----|
| X1 | 200 | 400 |
| X2 | 100 | 300 |
| X3 | 40 | 72 |
| X4 | 5 | 21 |
| X5 | 65 | 89 |

An arithmetic sequence is formed based on the value range of each component in the basal culture medium formulation. In this example, an arithmetic sequence with a length of 5 (that is, M = 5) is formed, and the results are shown in Table 2 below. An arithmetic sequence with a length of M can be formed as actually required.

Table 2. Arithmetic Sequences of Component Concentration in Formulation

| X1 | 200 | 250 | 300 | 350 | 400 |
|----|-----|-----|-----|-----|-----|
| X2 | 100 | 150 | 200 | 250 | 300 |
| X3 | 40 | 48 | 56 | 64 | 72 |
| X4 | 5 | 9 | 13 | 17 | 21 |
| X5 | 65 | 71 | 77 | 83 | 89 |

The data of the arithmetic sequence matrix is disorderly sorted to form a new matrix as shown in Table 3 below.

Table 3. Disorderly Sorting Results of Arithmetic Sequence of Component Concentration in Formulation

| X1 | 350 | 250 | 200 | 400 | 300 |
|----|-----|-----|-----|-----|-----|
| X2 | 150 | 250 | 300 | 200 | 100 |
| X3 | 48 | 56 | 64 | 72 | 40 |
| X4 | 9 | 5 | 21 | 17 | 13 |
| X5 | 89 | 77 | 83 | 35 | 71 |

The matrix formed by disorderly sorting are transposed to obtain M sets of new formulations, wherein M is the length of the arithmetic sequence. In this example, the length of the arithmetic sequence is 5, so that 5 sets of new formulations are obtained, as shown in Table 4 below.

Table 4. 5 Sets of Basal Culture Medium Formulations by Transposition

| Formulation No. | X1 | X2 | X3 | X4 | X5 |
|-----------------|-----|-----|----|----|----|
| No.1 | 350 | 150 | 48 | 9 | 89 |
| No.2 | 250 | 250 | 56 | 5 | 77 |
| No.3 | 200 | 300 | 64 | 21 | 83 |
| No.4 | 400 | 200 | 72 | 17 | 13 |
| No.5 | 300 | 100 | 40 | 65 | 71 |

The purpose of disorderly sorting and transposition of an arithmetic sequence matrix is to form different formulations, which can be disorderly sorted and transposed for several times to form more formulations. In this example, the formulations formed after disorderly sorting and transposition for twice are shown in Table 5 below.

Table 5. Basal Culture Medium Formulations Obtained after Disorderly Sorting and Transposition of Arithmetic Sequence Matrix for Twice

| Formulation No. | X1 | X2 | X3 | X4 | X5 |
|-----------------|-----|-----|----|----|----|
| No.1 | 350 | 150 | 48 | 9 | 89 |
| No.2 | 250 | 250 | 56 | 5 | 77 |
| No.3 | 200 | 300 | 64 | 21 | 83 |
| No.4 | 400 | 200 | 72 | 17 | 65 |
| No.5 | 300 | 100 | 40 | 13 | 71 |
| No.6 | 200 | 300 | 72 | 21 | 83 |

(continued)

| Formulation No. | X1 | X2 | X3 | X4 | X5 |
|---|---|---|---|---|---|
| No.7 | 400 | 100 | 48 | 5 | 89 |
| No.8 | 350 | 150 | 40 | 17 | 71 |
| No.9 | 300 | 250 | 56 | 13 | 77 |
| No.10 | 250 | 200 | 64 | 9 | 65 |

The underlined parts in Table 5 are the formulations formed after the second disorderly sorting and transposition. After disorderly sorting and transposition for K times, K*M sets of formulations can be formed, wherein M is the length of an arithmetic sequence.

A large number of basal culture medium formulations are obtained by the above method, and each output indicator for all formulations are predicted using the optimal formulation prediction model. In this example, a total of 900,000 sets of basal culture medium formulations are formed, and then the formulation prediction model is adopted to predict the output indicator, i.e., cell density. After sorting the prediction results from high to low, the top 10 formulations are selected and recommended as recommended basal culture medium formulations. The prediction results are shown in Table 6 below (only concentrations of 10 components are listed in the table).

Table 6. Data Table of Predicted Optimal 10 Formulations

| Components No. | L-glutamic acid | L-alanine | L-tryptophan | manganese sulfate monohydrate | sodium selenite | cobalt chloride hexahydrate | pyridoxal hydrochloride | sodium pyruvate | HEPES(pH buffered reagents) | sodium bicarbonate | predicted value (maximum cell density) |
|---|---|---|---|---|---|---|---|---|---|---|---|
| 1 | 283.33 | 40.00 | 188.89 | 0.00138 | 0.086 | 0.063 | 1.7 | 367 | 1344 | 2100 | 1.65E+07 |
| 2 | 244.44 | 10.00 | 260.00 | 0.00246 | 0.040 | 0.010 | 1.7 | 256 | 2067 | 2167 | 1.62E+07 |
| 3 | 400.00 | 10.00 | 135.56 | 0.01000 | 0.086 | 0.250 | 5.0 | 422 | 1778 | 2300 | 1.39E+07 |
| 4 | 361.11 | 50.00 | 260.00 | 0.00892 | 0.313 | 0.223 | 5.7 | 450 | 2067 | 1967 | 1.33E+07 |
| 5 | 400.00 | 60.00 | 135.56 | 0.00569 | 0.359 | 0.090 | 3.0 | 367 | 1633 | 1900 | 1.32E+07 |
| 6 | 283.33 | 20.00 | 117.78 | 0.00784 | 0.040 | 0.037 | 3.7 | 394 | 1344 | 2433 | 1.27E+07 |
| 7 | 361.11 | 50.00 | 188.89 | 0.00569 | 0.086 | 0.197 | 1.0 | 200 | 2500 | 2167 | 1.25E+07 |
| 8 | 244.44 | 20.00 | 188.89 | 0.01000 | 0.040 | 0.250 | 1.7 | 311 | 1200 | 2100 | 1.21E+07 |
| 9 | 244.44 | 90.00 | 153.33 | 0.00030 | 0.040 | 0.037 | 2.3 | 339 | 2356 | 2500 | 1.14E+07 |
| 10 | 244.44 | 60.0 | 260.00 | 0.00353 | 0.131 | 0.090 | 4.3 | 200 | 2356 | 2033 | 1.06E+07 |

(4) Conducting cell culture experiments with the recommended basal culture medium formulations obtained in step (3), to verify culture indicators of the recommended basal culture medium formulations and determine an optimal basal culture medium formulation accordingly.

Specifically, the candidate formulations are used for cell culture experiments, and one or more formulation(s) with the best values of Y1, Y2, ..., Ym are used as the optimal basal culture medium formulations based on the experimental results.

In this example, cell culture mediums are prepared based on the 10 optimal formulations recommended by AI, and cell culture experiments are conducted to verify the optimal formulations. The experimental results are shown in table 7 below.

Table 7. Cell Culture Results of 10 Formulations Recommended by AI

| Components No. | L-glutamic acid | L-alanine | L-tryptophan | manganese sulfate monohydrate | sodium selenite | cobalt chloride hexahydrate | pyridoxal hydrochloride | sodium pyruvate | HEPES | sodium bicarbonate | predicted value (maximum cell density) |
|---|---|---|---|---|---|---|---|---|---|---|---|
| 1 | 283.33 | 40.00 | 188.89 | 0.00138 | 0.086 | 0.063 | 1.7 | 367 | 1344 | 2100 | 1.03E+07 |
| 2 | 244.44 | 10.00 | 260.00 | 0.00246 | 0.040 | 0.010 | 1.7 | 256 | 2067 | 2167 | 8.16E+06 |
| 3 | 400.00 | 10.00 | 135.56 | 0.01000 | 0.086 | 0.250 | 5.0 | 422 | 1778 | 2300 | 1.40E+07 |
| 4 | 361.11 | 50.00 | 260.00 | 0.00892 | 0.313 | 0.223 | 5.7 | 450 | 2067 | 1967 | 3.00E+06 |
| 5 | 400.00 | 60.00 | 135.56 | 0.00569 | 0.359 | 0.090 | 3.0 | 367 | 1633 | 1900 | 8.60 E+06 |
| 6 | 283.33 | 20.00 | 117.78 | 0.00784 | 0.040 | 0.037 | 3.7 | 394 | 1344 | 2433 | 1.31E+07 |
| 7 | 361.11 | 50.00 | 188.89 | 0.00569 | 0.086 | 0.197 | 1.0 | 200 | 2500 | 2167 | 5.22E+06 |
| 8 | 244.44 | 20.00 | 188.89 | 0.01000 | 0.040 | 0.250 | 1.7 | 311 | 1200 | 2100 | 8.13E+06 |
| 9 | 244.44 | 90.00 | 153.33 | 0.00030 | 0.040 | 0.037 | 2.3 | 339 | 2356 | 2500 | 1.25E+07 |
| 10 | 244.44 | 60.0 | 260.00 | 0.00353 | 0.131 | 0.090 | 4.3 | 200 | 2356 | 2033 | 7.05E+06 |

The actual values and predicted values of the 10 optimal formulations are compared, and it is found that the predicted values of Formulations Nos. 3, 6, and 9 are close to the actual values, with high cell density, which can meet the requirements. Therefore, the three medium culture formulations are selected as the optimal basal culture medium formulations, and the finally resultant optimal formulations and culture results are shown in Table 8 below.

Table 8. Optimal Basal Culture Medium Formulations

| Components No. | L-glutamic acid | L-alanine | L-tryptophan | manganese sulfate monohydrate | sodium selenite | cobalt chloride hexahydrate | pyridoxal hydrochloride | sodium pyruvate | HEPES | sodium bicarbonate | predicted value (maximum cell density) | Actual value (maximum cell density) |
|---|---|---|---|---|---|---|---|---|---|---|---|---|
| 3 | 400.00 | 10.00 | 135.56 | 0.01000 | 0.086 | 0.250 | 5.0 | 422 | 1778 | 2300 | 1.39E+07 | 1.40E+07 |
| 6 | 283.33 | 20.00 | 117.78 | 0.00784 | 0.040 | 0.037 | 3.7 | 394 | 1344 | 2433 | 1.27E+07 | 1.31E+07 |
| 9 | 244.44 | 90.00 | 153.33 | 0.00030 | 0.040 | 0.037 | 2.3 | 339 | 2356 | 2500 | 1.14E+07 | 1.25E+07 |

It is readily understood by those of ordinary skill in the art that the above is only an optimal example of the present application and is not intended to limit the present application. Any modification, equivalent substitution, improvement, etc. all fall within the protection scope of the present application.

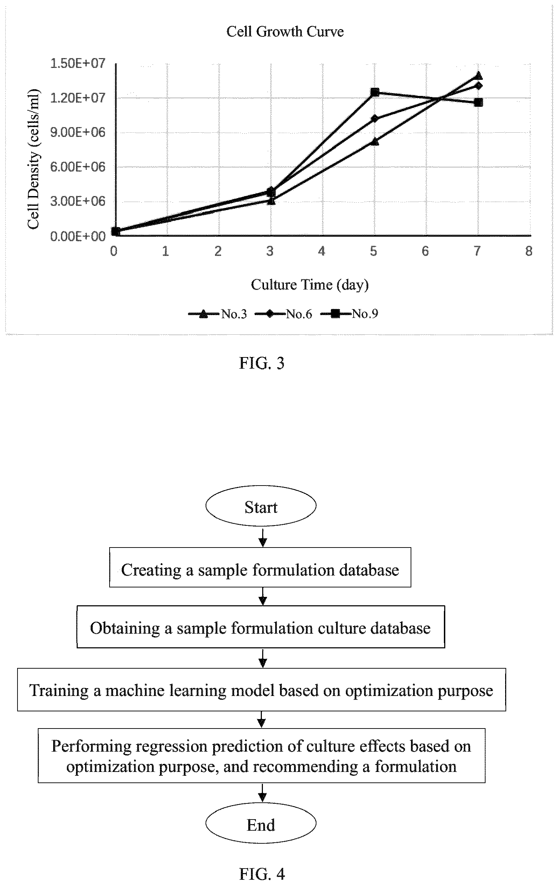

The present application provides a method for developing a basal culture medium formulation based on artificial intelligence, as shown in FIG.4, comprising the following steps.

(1) Creating a sample formulation database. A search space of addition proportion for each component in a basal culture medium formulation to be developed is determined, a basal culture medium sample formulation is formed by searching within the search space for each component, and a sample formulation database is created by collecting the basal culture medium sample formulations. The addition proportion for a component is a ratio of addition value to the maximum addition value of the component, its search space is in a range from the lowest addition proportion to 100%, and the lowest addition proportion is a ratio of the minimum addition value to the maximum value of the component.

The step of forming a basal culture medium sample formulation by searching within the search space for each component includes, but is not limited to, the following four methods, i.e., random generation of formulation, DOE design of experiment formulation, formulation formation by mixing, and a historical AI recommendation of formulation.

The random generation of formulation includes forming a base cultural medium sample formulation by randomly taking values within the search space for each component in the basal culture medium formulation.

The DOE design of experiment formulation includes the following step of:

- S1, clustering the lowest addition proportion of each component in the basal culture medium to obtain multiple addition magnitudes, and dividing each component in the basal culture medium into functional categories based on its functions, wherein the functional categories include amino acids, trace metal ions, vitamins, lipids, buffered reagents, and the like; and

- S2, forming a DOE experimental factor by combining the multiple addition magnitudes and functional categories obtained in step S1, and forming a basal sample formulation by using space-filling DOE experimental design, wherein the space-filling DOE experimental design is a ball filling method, a Latin hypercube method, a uniform method or a minimum potential method, and preferably a Latin hypercube method.

The formulation formation by mixing includes obtaining a new basal culture medium sample formulation by screening and combining existing basal culture medium sample formulation. Preferably, the step of screening and combining existing basal culture medium sample formulation includes, verifying culture effects of the existing basal culture medium sample formulations, selecting the formulations with a high cell survival rate, a high cell density, or a high protein expression level, and forming a new formulation by mixing the above two or three or more formulations in random or preset proportions.

The historical AI recommendation of formulation includes developing and obtaining a basal culture medium formulation based on artificial intelligence according to the method for developing a formulation provided in the present application.

The quality and number of basal culture medium sample formulations are the key and premise of artificial intelligence to optimize culture effects of a basal culture medium. Hundreds of basal culture medium sample formulations are required for machine learning to perform more accurate model training. Moreover, basal culture medium sample formulations should cover known or unknown different dimensions and different effects, and more basal culture medium sample formulations are distributed in the high- dimensional space region with better effects or the high-dimensional space region with obvious changes. Therefore, in order to increase the number, and to more intensively select basal culture medium sample formulations from the high-dimensional space region with better effects or the high-dimensional space region with obvious changes, the DOE design of experiment formulation is preferable in the present application. Moreover, in order to improve coverage of the sample formulations with different dimensions and different effects, randomly generated basal culture medium sample formulations are added, assisting with formulation formation by mixing. It is shown in the experiments that a sample formulation database created in combination with the above three methods has better effects of machine learning model training.

Preferably, a ratio of the number of randomly generated formulations to the number of DOE designed formulations in the sample formulation database is 1~4:10. The generalization ability of the method is ensured by random generation of formulation, and the prediction accuracy of the method is better improved by DOE design of experiment formulations.

The total number of the samples in the sample formulation database is 100 or more, which can realize the method for developing basal culture medium formulations based on artificial intelligence provided in the present application. Preferably, the total number of the samples in the sample formulation database is 1000 or more, including 100 to 200 of randomly generated formulations and 50 to 200 of DOE designed formulations, so as to control time cost for preparation of culture mediums. Since the preparation and culture effects verification would inevitably be performed with historical AI recommended formulation in the process of experiments, the cost for preparation of culture mediums and culture effects verification is not additionally increased. The rest are mixed forming formulations, and time cost for preparation of the mediums can be greatly reduced since the prepared mediums are mixed.

(2) Acquiring a sample formulation culture database. Culture effects of each basal culture medium sample formulation stored in the sample formulation database obtained in the step (1) are acquired by conducting experimental verification based on optimization purpose, and basal culture medium sample formulation data associated with culture effects are collected as a sample formulation culture database.

Specifically, the step of acquiring culture effects of each basal culture medium sample formulation by conducting experimental verification based on optimization purpose includes, culturing target cells by using the basal culture medium sample formulation, and sampling and detecting cell states at time points during the culture process, wherein the cell states include a cell survival rate, a cell density, and/or biochemical indicators, and the biochemical indicators are a protein expression level, glucose content, lactic acid content, ammonia content, and/or glutamine content; and fitting the cell survival rate to obtain a cell survival rate curve of the basal culture medium sample formulation with respect to culture time, and fitting the cell density to obtain a cell growth curve of the basal culture medium sample formulation with respect to culture time, wherein culture effects of the basal culture medium sample formulation is one or more (corresponding to the use of multi-objective optimization of a machine learning model) or a combination (for example, a weighted sum of specific indicators) of the cell growth curve with respect to culture time, the cell survival rate curve with respect to culture time, or the cell density, the cell survival rate, or the biochemical indicators at a specific time point of the basal culture medium.

(3) Training a machine learning model by the sample formulation culture database obtained in step (2) based on optimization purpose, to obtain a model for predicting culture effects of a basal culture medium formulation.

The machine learning model includes, but is not limited to, a support vector machine regression model, a K-nearest neighbor model, XGBoost, a ridge regression model, LightGBM, a random forest model, GBDT, or a deep learning model, wherein the deep learning model includes, but is not limited to, a fully connected neural network, a convolution neural network, or a recurrent neural network.

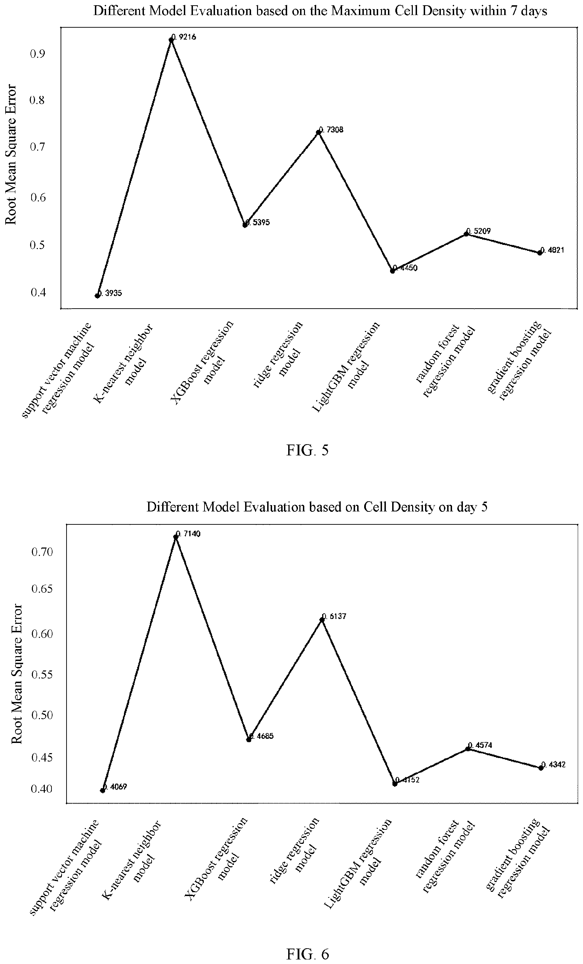

The support vector machine regression model has better effects. It is found based on 15-fold cross-validation that, as shown in FIGs. 5 and 6, the support vector machine regression model has better model performances, and the model is a continuous differentiable model, which also has a greater advantage in recommending an optimal formulation. The other machine learning models, such as the K-nearest neighbor model, or the ridge regression model, are less accurate on test sets. In addition, the machine learning models based on tree structures such as XGBoost, LightGBM, the random forest model, or GBDT, are not continuous differentiable models. The deep learning model includes, such as a fully connected neural network, a convolutional neural network, a recurrent neural network, or the like. The convolutional neural network is suitable for data with translational invariance, such as image processing. The recurrent neural network is suitable for sequential data, such as speech text processing, but has no particular advantage in predicting optimal formulations. Moreover, the deep learning model requires a lot of data and costs more. Therefore, the support vector machine regression model with the lowest root mean square error is the preferred model.

(4) Performing regression prediction of culture effects by adopting the model for predicting culture effects of a basal culture medium formulation obtained in step (3) within the search space of addition proportion for each component in the basal culture medium formulation to be optimized based on development purpose, and preferentially recommending a basal culture medium formulation based on the predicted culture effects.

Specifically, a global optimization algorithm or a heuristic algorithm can be used for regression prediction of culture effects by searching a basal culture medium formulation within the search space. The heuristic algorithm includes, but is not limited to, a genetic algorithm, a greedy algorithm, an annealing algorithm, an ant colony algorithm, a particle swarm algorithm, an artificial bee colony algorithm, an artificial fish swarm algorithm, a shuffled frog leaping algorithm, a fireworks algorithm, a bacterial foraging optimization algorithm, or a firefly algorithm. The global optimization algorithm includes, but is not limited to, Newton's method, quasi-Newton method, a conjugate gradient method, or a gradient descent method commonly used for deep learning. Variants of a commonly used gradient descent method include SGD, Momentum, Adagrad, RMSprop, Adam, Nadam or the like.

Most heuristic algorithms, such as a genetic algorithm, an ant colony algorithm, a particle swarm algorithm, an artificial bee colony algorithm, an artificial fish swarm algorithm, a shuffled frog leaping algorithm, a fireworks algorithm, a bacterial foraging optimization algorithm, a firefly algorithm, or the like, require to take up a lot of buffer space and need a lot of computation in terms of space complexity. A greedy algorithm could not easily to obtain a global optimal value for the problem of recommending such a multi-factor interaction of multi-component content formulations. Moreover, an annealing algorithm can only use increment as an evaluation index, and is not comparable to a gradient descent method in which gradient is used as an evaluation index. In addition, the algorithms such as Newton's method, quasi-Newton method, or a conjugate gradient method can use the second-order gradient approximation, which is theoretically superior to the first-order gradient approximation of a gradient descent method, but computational burden of such methods is greater due to the complexity of the formulations. Therefore, various variants of the gradient descent method are preferrable.

The present application provides a system for developing a basal culture medium formulation based on artificial intelligence, comprising a sample formulation generation module, a sample formulation culture database, a regression model training module, and a formulation recommendation module.

The sample formulation generation module is configured for forming a basal culture medium sample formulation by searching within the search space of addition proportion for each component in the basal culture medium formulation to be optimized, and creating a sample formulation database.

The sample formulation culture database is configured for storing the data of each basal culture medium sample formulation and the associated culture effects in the sample formulation culture database.

The regression model training module is configured for selecting a regression model, and performing regression model training with the basal culture medium sample formulations and associated culture effects stored in the sample formulation culture database, to obtain and store a model for predicting culture effects of a basal culture medium formulation.

The formulation recommendation module is configured for predicting culture effects of a basal culture medium formulation within the search space by adopting the model for predicting culture effects of a basal culture medium formulation stored in the regression model training module, and preferentially recommending a basal culture medium formulation accordingly.

The following method is shown as an example.

A method for developing a basal culture medium formulation based on artificial intelligence, taking a small number of formulation components as an example, comprises the steps of

(1) Creating a sample formulation database of L-tryptophan, L-cysteine, L-glycine, L-alanine, manganese sulfate monohydrate, cobalt chloride hexahydrate, pyridoxal hydrochloride, ethanolamine, sodium bicarbonate, and poloxamer 188, determining a search space of addition proportion for each component, forming a basal culture medium sample formulation by searching within the search space for each component, and collecting basal culture medium sample formulations to create a sample formulation database. The addition proportion of a component is a ratio of addition value to the maximum addition value of the component, its search space is in a range from the lowest addition proportion to 100%, and the lowest addition proportion is a ratio of the minimum addition value to the maximum value of the component.

The step of forming a training formulation by searching within the search space for each component includes the following four methods, i.e., random generation of formulation, DOE design of experiment formulation, formulation formation by mixing, and a historical AI recommendation of formulation.

The random generation of formulation includes forming a basal cultural medium sample formulation by randomly taking values within the search space for each component in the basal culture medium formulation.

In this example, the DOE design of experiment formulation is used as follows.

Specifically, except for a few unchanging components such as glucose, all the other components in the formulation are divided into five major categories, such as amino acids, trace metal ions, vitamins, lipids, buffered reagents, etc. In each major category, each component has a maximum addition value of 100%, and a lowest addition percentage is obtained from dividing a minimum value by a maximum value in the formulation. The components with the lowest addition percentage close to each other are selected to form a new category, so that nine major categories, i.e., nine factors, are formed based on the five major categories. Ninety formulations are designed using a space-filling Latin hypercube DOE design of experiment.

The formulation formation by mixing used in this example specifically comprises verifying culture effects of the existing basal culture medium sample formulation, and selecting and mixing two or three or more of the above formulations with a high cell viability, a high cell density, or a high protein expression level in a random ratio to form a new formulation.

The historical AI recommendation of formulation includes optimizing and obtaining a basal culture medium formulation according to the method for optimizing formulation provided in the present application based on artificial intelligence.

The sample formulation database finally created in this example includes 1000-1500 of basal culture medium formulations, including 90 of DOE designed formulations, 200 of randomly generated formulations, 100-200 of historical AI recommended formulations, and the rest is 500-700 of mixed forming formulations.

(2) Obtaining a sample formulation culture database. Culture effects of each basal culture medium sample formulation stored in the sample formulation database obtained in step (1) are acquired by conducting experimental verification based on optimization purpose, and basal culture medium sample formulation data associated with the culture effects are collected as a sample formulation culture database. In this example, the step of acquiring culture effects of each basal culture medium sample formulation by conducting experimental verification based on optimization purpose includes the following steps.

Specifically, a batch culture is performed by inoculating cells with a cell density of $0.5 \times 10^6$ cells/mL, and a culture volume is 10 mL, a culture vessel is 50mL mini bioreactor, the rotation speed of the shaker was 180 rpm, and the culture time is 7 days. During the culture process, samples are taken on day 3, day 5, and day 7, respectively. The cell density is counted, and the biochemical parameters such as glucose, lactic acid, ammonia, glutamine, and protein expression levels are measured. Glucose is supplemented to 4~5g/L according to the consumption of glucose. To obtain complete test data, samples are taken every day.

When AI models are built, single-target modeling or multi-target modeling can be performed using only cell survival rate, cell density, or biochemical parameters at one sampling point. Regression models can also be built using a cell survival rate curve or a cell density curve.

The following data are obtained in this example, i.e., a cell density at each sampling point, the maximum cell density within 7 days, and a cell growth curve plotted using cell density data.

Relevant data such as the content of each component in the formulation, the data during the preparation of the culture medium, the culture effects, or the like, are recorded and stored in the culture database.

(3) Training a regression model by using the sample formulation culture database obtained in step (2) to obtain a model for predicting culture effects of a basal culture medium formulation.

Specifically, a support vector machine regression model is loaded using the python language, and 15-fold cross-validation is performed using the RBF (Gaussian) kernel function. Formulation culture effect prediction model 1 is obtained for the maximum cell density within 7 days, with retaining an average root mean square error of two decimal places, i.e., approximately equaling to 0.39. Formulation culture effect prediction model 2 is obtained for the cell density on day 5, with retaining an average root mean square error of two decimal places, i.e., approximately equaling to 0.41. The model is intended to perfectly predict yield and quality of the cells cultured at different levels of each component in a culture medium formulation.

If the training results could not meet the criteria, steps (1) and (2) are repeated to increase the training sample data size.

(4) Performing regression prediction of culture effects by adopting the model for predicting culture effects of a basal culture medium formulation obtained in step (3) within the search space of addition proportion for each component in the basal culture medium formulation to be optimized based on optimization purpose, and preferentially recommending a basal culture medium formulation based on the predicted culture effects.

Specifically in this example, the cell culture effects most likely to occur for each component at different contents is simulated in computer numerically by depth dig for model information based on the above maximum cell density within 7 days and cell density on day 5. A gradient descent method is used for simulation calculation, with the gradient, i.e., the effect of increasing each component at a specific content by one unit on the change in cell culture effects, being divided into positive and negative. A positive gradient indicates that the increase in the content of a component is beneficial to the cell culture effects, and a negative gradient indicates that the increase in the content of a component is detrimental to the cell culture effects. Based on the above simulation results, the content of a component is gradually modified by increasing the content of a component if it is a positive gradient and decreasing the content of a component if it is a negative gradient, with the increased or decreased content of a component is proportional to the gradient value. Then simulation calculation is repeated, and the component is adjusted based on the gradient. The above steps are repeated until it is found in the numerical simulation that the gradient is infinitely close to zero, and that culture effects of the cell simulation could not be further improved by modifying the content of a component, and the culture medium formulation is the optimal formulation under model simulation. The above gradient descent method has an algorithmic form of SGD, Momentum, Adagrad, RMSprop, Adam, or the like.

By successfully building the above machine learning model, the effect of each component on culture effects of cells can be successfully measured. If a machine learning model fails to converge or has the problem of low accuracy, poor generalization ability, or the like during training, the data would be judged to be insufficient, and steps (1) to (3) are repeated to continue to form more basal culture medium sample formulations by random generation, DOE design of experiment, or formulation mixing, to expand the data, and optimize the machine learning model.

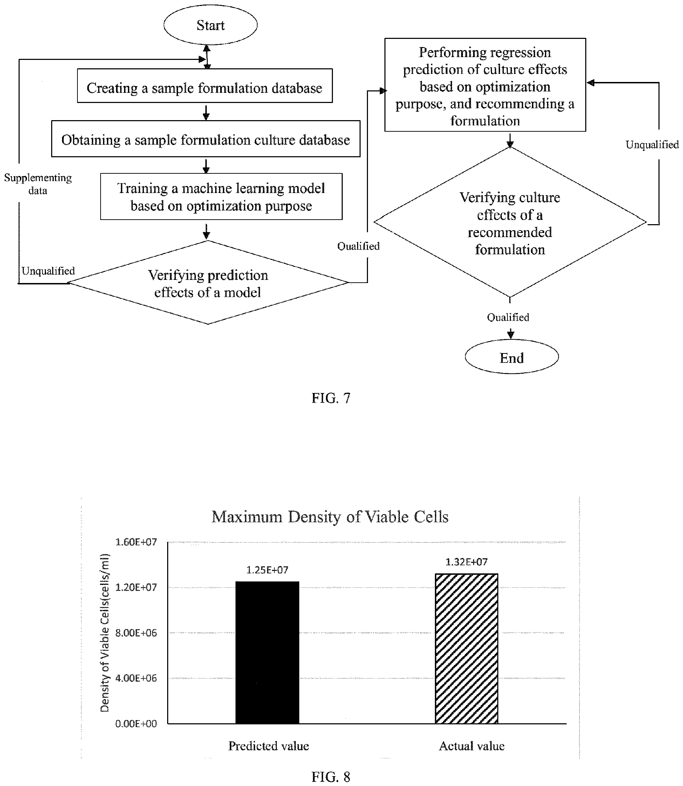

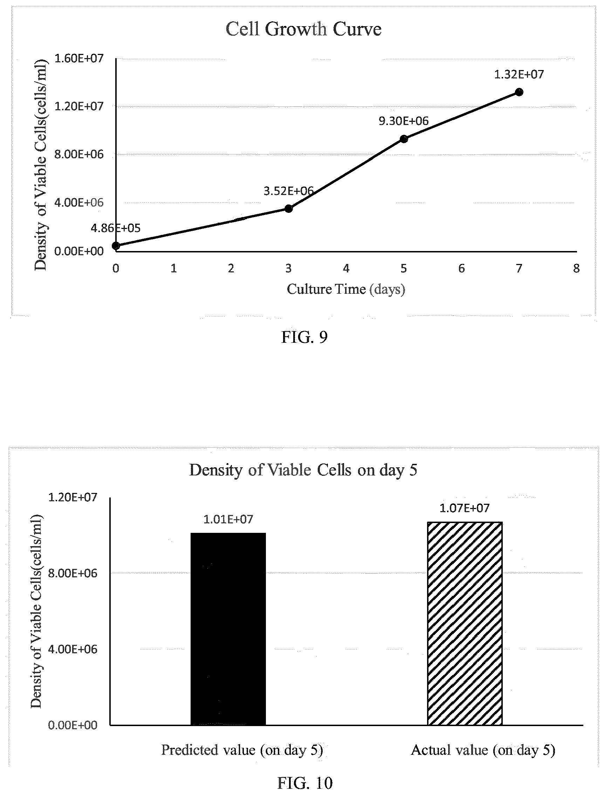

The optimal formulation recommended by the above numerical simulation after machine learning includes specifying the content of each component in detail, preparing a culture medium according to the formulation, and performing a batch culture experiment. If culture effects of the cell culture could not meet the experimental requirements, step (4) is repeated. After building a model by the above machine learning, the recommended formulation after adjusting the components is tested and a basal culture medium formulation is generated with respect to the maximum cell density within 7 days, respectively. Then the medium is inoculated with cells for a batch culture. The cell survival rate is kept stable, the 7-day mortality rate is 0%, and the cell density is extremely high. The predicted value of the model is close to the actual value of the model, and the predicted formulation is reliable. The specific formulation data is shown in the following table, the comparison of the predicted value with the actual value of the cultured cell is shown in FIG. 8 and the cell growth curve is shown in FIG. 9.

| Components | Concentration (mg/L) |
|---|---|
| L-tryptophan | 156.41 |
| L-cysteine | 165.04 |
| L-glycine | 99.31 |
| L-alanine | 58.06 |
| manganese sulfate monohydrate | 0.00373 |
| cobalt chloride hexahydrate | 0.108 |
| pyridoxal hydrochloride | 3.20 |
| ethanolamine | 3.17 |
| sodium bicarbonate | 2459 |
| poloxamer 188 | 1396 |

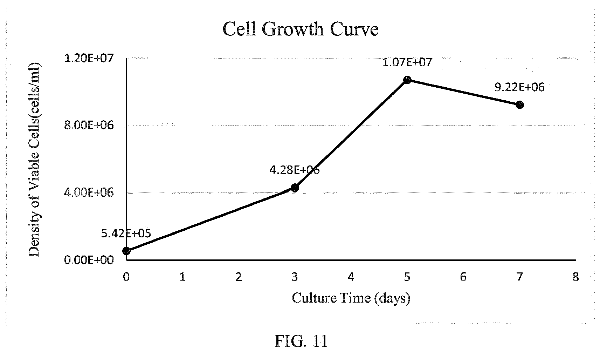

A basal culture medium formulation is generated with respect to the cell density on day 5. Then the medium is inoculated with cells for a batch culture. The cell survival rate is kept stable, the 7-day mortality rate is 0%, and the cell density is extremely high. The predicted value of the model is close to the actual value of the model, and the predicted formulation is reliable. The specific formulation data is shown in the following table, the comparison of the predicted value with the actual value of the cultured cell is shown in FIG. 10, and the cell growth curve is shown in FIG. 11.

| Components | L-tryptophan | L-cysteine | L-glycine | L-alanine | manganese sulfate monohydrate | cobalt chloride hexahydrate | pyridoxal hydrochloride | ethanolamine | sodium bicarbonate | poloxamer 188 |
|---|---|---|---|---|---|---|---|---|---|---|
| Concentration (mg/L) | 202.38 | 147.80 | 93.44 | 52.41 | 0.00579 | 0.126 | 0.006 | 4.90 | 2208 | 1052 |

The formulation optimization period in this example is about 5 months if the creation of sample formulation culture database (more than 1000 formulations) and machine learning model training are included. When it is used, it only takes half a month to develop a formulation by performing learning model training, formulation recommendation and effect verification, which can greatly shorten the development period of a basal culture medium and make it easier to develop a basal culture medium.

It is readily understood by those of ordinary skill in the art that the above is only an optimal example of the present application and is not intended to limit the present application. Any modification, equivalent substitution, improvement, etc. all fall within the protection scope of the present application.

**Claims**

1. A method for developing a basal culture medium, comprising the following steps of

   - selecting and optimizing regression models for selected culture indicators through an experimentally verified basal culture medium formulation database, to determine a regression prediction model for predicting the culture indicators of a basal culture medium;
   - acquiring an addition range of each component in the basal culture medium, and enumerating and randomly selecting addition amount of each component, to generate a plurality of candidate basal culture medium formulations;
   - predicting the culture indicators of the obtained candidate basal culture medium formulations by adopting the regression prediction model, and screening one or more formulations from the candidate basal culture medium formulations as recommended basal culture medium formulations based on prediction results; and
   - conducting cell culture experiments with the obtained recommended basal culture medium formulations, to verify the culture indicators of the recommended basal culture medium formulations and determine an optimal basal culture medium formulation based on the verified culture indicators.

2. The method of claim 1, wherein the step of determining a regression prediction model for predicting the culture indicators of a basal culture medium, comprises

   - forming a training formulation by searching within the addition range of each component in the basal culture medium;
   - acquiring culture indicator data of the training formulation by conducting cell culture experiments;
   - creating a training sample data set with the addition amount of each component or its normalized value in the training formulation as an input matrix and the culture indicator data as an output matrix; and
   - training and testing multiple alternative regression prediction models with the training sample data set under the same conditions, and selecting one of the trained and tested multiple alternative regression prediction models as the regression prediction model for predicting the culture indicators of a basal culture medium.

3. The method of claim 2, wherein the culture indicators comprise a cell survival rate, a cell density, a protein expression level, glucose, lactic acid, or ammonia.

4. The method of claim 2, wherein the step of creating a training sample data set with the addition amount of each component or its normalized value in the training formulation as an input matrix and the culture indicator data as an output matrix, comprises

   - forming a set of experimental data by input data $(x_1, x_2, ..., x_n)$ and output data $(y_1, y_2, ..., y_m)$; wherein $x_i$ is the $i^{th}$ component of the basal culture medium formulation, used as a feature during model training, verification and testing; $y_1$ represents a cell survival rate, $y_2$ represents a cell density, $y_3$ represents a protein expression level, ..., and $y_m$ represents the $m^{th}$ output indicator; the input matrix of the regression model is X matrix, wherein $x_{ij}$ represents the $j^{th}$ component of the $i^{th}$ formulation; and the output matrix is Y matrix, wherein $y_{ij}$ represents the $j^{th}$ output value of the $i^{th}$ formulation:

$$X = \begin{pmatrix} x_{11} & x_{12} & \cdots & x_{1n} \\ \vdots & \vdots & \cdots & \vdots \\ x_{n1} & x_{n2} & \cdots & x_{nn} \end{pmatrix}$$

$$Y = \begin{pmatrix} y_{11} & y_{12} & \cdots & y_{1m} \\ \vdots & \vdots & \cdots & \vdots \\ y_{n1} & y_{n2} & \cdots & y_{nm} \end{pmatrix}$$

.

**5.** The method of claim 2, wherein the step of creating a training sample data set with the addition amount of each component or its normalized value in the training formulation as an input matrix and the culture indicator data as an output matrix, comprises

- optimizing the components in the training formulation through feature selection of the regression model.

**6.** The method of claim 2, wherein the multiple alternative regression prediction models comprise a support vector regression model, an elastic network model, an Xgboost model, a Gradient Boosting Regression model, a Logostic Regression model, a regression model based on multi-layer neural network, a regression model based on convolution neural network, and a regression model based on recurrent neural network.

**7.** The method of claim 1, wherein the step of acquiring an addition range of each component in the basal culture medium, and enumerating and randomly selecting addition amount of each component to generate a plurality of candidate basal culture medium formulations, comprises:

- acquiring a point value with a same number within a value range of each component in the basal culture medium, and forming a value sequence of each component;
- sorting the value sequences of all components, to obtain a rearranged component value sequence; and
- constructing a component value matrix by taking the rearranged component value sequence as a line or a column, and obtaining candidate basal culture medium formulations by taking the column or the line of the component value matrix as a value of each component.

**8.** The method of claim 7, wherein the candidate basal culture medium formulations have a number of 1, 000 ~1, 000, 000.

**9.** A system for developing a basal culture medium formulation, comprising:

- a regression model selection module, configured for selecting and optimizing a regression model of selected culture indicators through an experimentally verified basal culture medium formulation database, to determine a regression prediction model for predicting the culture indicators of a basal culture medium;
- a candidate basal culture medium formulation generation module, configured for acquiring an addition range of each component in the basal culture medium, and enumerating and randomly selecting addition amount of each component, to generate a plurality of candidate basal culture medium formulations;
- a basal culture medium formulation recommendation module, configured for predicting the culture indicators of the obtained candidate basal culture medium formulations by adopting the regression prediction model, and screening one or more formulations from the candidate basal culture medium formulations as recommended basal culture medium formulations based on prediction results; and
- an optimal basal culture medium formulation determination module, configured for conducting cell culture experiments with the obtained recommended basal culture medium formulations, to verify the culture indicators of the recommended basal culture medium formulations and determine an optimal basal culture medium formulation based on the verified culture indicators.

**10.** The system of claim 9, wherein the candidate basal culture medium formulation generation module comprises

- an enumeration sub-module, configured for acquiring a point value with a same number within a value range of each component in the basal culture medium, and forming a value sequence of each component;
- a rearrangement sub-module, configured for sorting the value sequences of all components, to obtain a rearranged component value sequence; and
- a combination sub-module, configured for constructing a component value matrix by taking the rearranged component value sequence as a line or a column, and obtaining candidate basal culture medium formulations by taking the column or the line of the component value matrix as a value of each component.

**11.** A method for developing a basal culture medium formulation, comprising the following steps of

- acquiring components in candidate basal culture medium formulations, determining a search space of addition proportion for each component, forming basal culture medium sample formulations by searching within the search space for each component, and creating a sample formulation database by collecting the basal culture medium sample formulations;
- acquiring culture effects of each basal culture medium sample formulation stored in the sample formulation database by conducting experimental verification based on development purpose, and collecting basal culture medium sample formulation data associated with the culture effects as a sample formulation culture database;
- training a machine learning model by using the sample formulation culture database based on development purpose, and obtaining a model for predicting culture effects of a basal culture medium formulation; and
- performing regression prediction of culture effects by adopting the model for predicting culture effects of a basal culture medium formulation within the search space of addition proportion for each component in the basal culture medium formulation to be optimized based on development purpose, and recommending basal culture medium formulations based on the predicted culture effects.

**12.** The method of claim 11, wherein forming training basal culture medium sample formulations by searching within the search space for each component comprises random generation of formulation, DOE design of experiment formulation, formulation formation by mixing, and a historical AI recommended formulation.

**13.** The method of claim 12, wherein the random generation of formulation comprises forming basal culture medium sample formulations by randomly taking a value within the search space for each component in the basal culture medium formulation;

wherein the DOE design of experiment formulation comprises

- clustering the lowest addition proportion of each component in the basal culture medium formulations to obtain multiple addition magnitudes; and dividing each component in the basal culture medium formulations into functional categories based on its functions, wherein the functional categories comprise amino acids, trace metal ions, vitamins, lipids, and buffered reagents; and
- forming a DOE experimental factor by combining the multiple addition magnitudes and functional categories, and forming a basal sample formulation by using a space-filling DOE design of experiment, wherein the space-filling DOE design of experiment includes a ball filling method, a Latin hypercube method, a uniform method, and a minimum potential method;

wherein the mixed forming formulation comprises screening and combining existing basal culture medium sample formulations to obtain new basal culture medium sample formulations; including verifying culture effects of the existing basal culture medium sample formulation, selecting the formulations with a high cell survival rate, a high cell density or a high protein expression level, and forming the new formulations by mixing two or three or more of the formulations by random or preset proportions; and
wherein the historical AI recommendation of formulation comprises obtaining basal culture medium formulations by recommending based on an artificial intelligence method.

**14.** The method of claim 12, wherein a ratio of the number of randomly generated formulations to the number of DOE designed formulations in the sample formulation database is 1-4:10.

**15.** The method of claim 12, wherein the total number of samples in the sample formulation database is 1000 or more, comprising 100 to 200 of the randomly generated formulations, 50 to 200 of the DOE designed formulations, 100 to 200 of the historical AI recommended formulations, and the rest is the mixed forming formulations.

**16.** The method of claim 11, wherein the addition proportion is a ratio of addition value to the maximum addition value of the component, and the search space for each component is in a range from the lowest addition proportion to 100%, wherein the lowest addition proportion is a ratio of the minimum addition value to the maximum addition value of the component.

**17.** The method of claim 11, wherein the step of acquiring culture effects of each basal culture medium sample formulation by conducting experimental verification based on development purpose, comprising

- culturing target cells using the basal culture medium sample formulations, and sampling and detecting cell states at time points during the culture process, wherein the cell states include a cell survival rate, a cell density, and/or biochemical indicators, and the biochemical indicators are a protein expression level, content of glucose, content of lactic acid, content of ammonia content, and/or content of glutamine; and
- fitting the cell survival rate to obtain a cell survival rate curve of the basal culture medium sample formulation with respect to culture time, and fitting the cell density to obtain a cell growth curve of the basal culture medium sample formulation with respect to culture time; wherein culture effects of the basal culture medium sample formulation are one or more or a combination of the cell growth curve or the cell survival rate curve of the basal culture medium sample formulation with respect to culture time, or the cell density, the cell survival rate and the biochemical indicators at a specific time point.

18. The method of claim 11, wherein the machine learning model comprises a support vector machine regression model, a K-nearest neighbor model, XGBoost, a ridge regression model, LightGBM, a random forest model, GBDT, and a deep learning model; and the deep learning model comprises a fully connected neural network, a convolution neural network, and a recurrent neural network.

19. The method of claim 11, wherein a global optimization algorithm or a heuristic algorithm is used to search a basal culture medium formulation within the search space for regression prediction of culture effects; the heuristic algorithm comprises a genetic algorithm, a greedy algorithm, an annealing algorithm, an ant colony algorithm, a particle swarm algorithm, an artificial bee colony algorithm, an artificial fish swarm algorithm, a shuffled frog leaping algorithm, a fireworks algorithm, a bacterial foraging optimization algorithm, and firefly algorithm; and the global optimization algorithm includes Newton's method, the quasi-Newton method, a conjugate gradient method, and a gradient descent method for deep learning; wherein the gradient descent method is preferably SGD, Momentum, Adagrad, RMSprop, Adam, and Nadam.

20. A system for developing a basal culture medium formulation, comprising:

- a sample formulation generation module, configured for acquiring components in candidate basal culture medium formulations, determining a search space of addition proportion for each component, forming basal culture medium sample formulations by searching within the search space for each component, and creating a sample formulation database by collecting the basal culture medium sample formulations;
- a sample formulation culture generation module, configured for acquiring culture effects of each basal culture medium sample formulation stored in the sample formulation database by conducting experimental verification based on development purpose, and collecting basal culture medium sample formulation data associated with the culture effects as a sample formulation culture database;
- a regression model training module, configured for training a machine learning model by using the sample formulation culture database based on development purpose, and obtaining a model for predicting culture effects of a basal culture medium formulation; and
- a formulation recommendation module, configured for performing regression prediction of culture effects by adopting the model for predicting culture effects of a basal culture medium formulation within the search space of addition proportion for each component in the basal culture medium formulation to be optimized based on development purpose, and recommending basal culture medium formulations based on the predicted culture effects.

```
┌─────────────────────────┐          ┌─────────────────────────────┐
│  Determining a regression│          │   Enumerating and randomly  │
│   prediction model for   │          │  selecting to generate      │
│  selected culture        │          │  candidate                  │
│  indicators              │          │  basal culture medium       │
│                          │          │  formulations               │
└─────────────────────────┘          └─────────────────────────────┘
              │                                    │
              │        ┌──────────────────────┐    │
              └───────▶│ Recommending basal    │◀───┘
                       │ culture               │
                       │ medium formulations   │
                       └──────────────────────┘
                                  │
                                  ▼
                       ┌──────────────────────────┐
                       │  Performing cell culture  │
                       │ experiments to determine  │
                       │ optimal                   │
                       │ basal culture medium      │
                       │ formulations              │
                       └──────────────────────────┘
```

FIG. 1

```
                          ┌──────────┐
                          │  Start   │
                          └────┬─────┘
                               ▼
          ┌────────────────────────────────────────────┐
          │ Reading value range of each component in   │
          │ a basal culture medium formulation         │
          └────────────────────┬───────────────────────┘
                               ▼
          ┌────────────────────────────────────────────┐
          │ Constructing matrixes respectively named   │
          │ "arithmetic sequence matrix" and           │
          │ "formulation matrix"                       │
          └────────────────────┬───────────────────────┘
                               ▼
     ┌──────────────────────────────────────────────────────┐
     │ Forming an arithmetic sequence with a length of M     │◄─┐
     │ based on value range of each component, and adding     │  │
     │ the sequence into the arithmetic sequence matrix       │  │
     └────────────────────┬─────────────────────────────────┘  │
                          ▼                                     │
                    ╱───────────╲                          No   │
                  ╱ Whether the   ╲──────────────────────────────┘
                  ╲ analysis is    ╱
                    ╲ finished for ╱
                      ╲ all comp. ╱
                       ╲────┬────╱
                            │ Yes
                            ▼
     ┌──────────────────────────────────────────────────────┐
     │ Disorderly sorting and transposition the contents of  │◄─┐
     │ the arithmetic sequence matrix by line, and forming    │  │
     │ a new formulation data                                 │  │
     └────────────────────┬─────────────────────────────────┘  │
                          ▼                                     │
          ┌────────────────────────────────────────────┐       │
          │ Adding the contents of transposition matrix│       │
          │ into the formulation matrix                │       │
          └────────────────────┬───────────────────────┘       │
                               ▼                                │
                        ╱───────────╲                      No   │
                      ╱ Whether disorderly╲────────────────────┘
                      ╲ sorting and        ╱
                        ╲ transposition is ╱
                          ╲ finished for N ╱
                            ╲ times...    ╱
                              ╲────┬────╱
                                   │ Yes
                                   ▼
          ┌────────────────────────────────────────────┐
          │ Submitting formulation data in the         │
          │ formulation matrix                         │
          └────────────────────┬───────────────────────┘
                               ▼
                          ┌──────────┐
                          │   End    │
                          └──────────┘
```

FIG. 2

## Cell Growth Curve

FIG. 3

FIG. 4

Different Model Evaluation based on the Maximum Cell Density within 7 days

FIG. 5

Different Model Evaluation based on Cell Density on day 5

FIG. 6

Start

Creating a sample formulation database

Obtaining a sample formulation culture database

Supplementing data

Training a machine learning model based on optimization purpose

Unqualified

Verifying prediction effects of a model

Qualified

Performing regression prediction of culture effects based on optimization purpose, and recommending a formulation

Unqualified

Verifying culture effects of a recommended formulation

Qualified

End

FIG. 7

## Maximum Density of Viable Cells

1.60E+07

1.25E+07

1.32E+07

1.20E+07

8.00E+06

4.00E+06

0.00E+00

Density of Viable Cells(cells/ml)

Predicted value

Actual value

FIG. 8

FIG. 9

FIG. 10

FIG. 11

## INTERNATIONAL SEARCH REPORT

| International application No. |
|---|
| **PCT/CN2021/131105** |

**A. CLASSIFICATION OF SUBJECT MATTER**

G16B 50/00(2019.01)i; G16C 20/70(2019.01)i

According to International Patent Classification (IPC) or to both national classification and IPC

**B. FIELDS SEARCHED**

Minimum documentation searched (classification system followed by classification symbols)

G16B; G16C

Documentation searched other than minimum documentation to the extent that such documents are included in the fields searched

Electronic data base consulted during the international search (name of data base and, where practicable, search terms used)

CNPAT, WPI, EPODOC, IEEE, ISI, CNKI: 培养基, 配方, 指标, 回归, 归一化, 模型, 训练, 预测, 矩阵, 输入, 输出, 样本, culture, medium, formula, index, regression, normalization, model, training, prediction, matrix, input, output, sample

**C. DOCUMENTS CONSIDERED TO BE RELEVANT**

| Category* | Citation of document, with indication, where appropriate, of the relevant passages | Relevant to claim No. |
|---|---|---|
| PX | CN 113450868 A (DONGGUAN TAILI BIOLOGICAL ENGINEERING CO., LTD.) 28 September 2021 (2021-09-28) claims 1-10, description paragraphs [0004]-[0156] | 1-20 |
| PX | CN 113450882 A (DONGGUAN TAILI BIOLOGICAL ENGINEERING CO., LTD.) 28 September 2021 (2021-09-28) claims 1-10 | 11-20 |
| X | CN 106096077 A (XINXIANG MEDICAL UNIVERSITY) 09 November 2016 (2016-11-09) description abstract, description paragraphs [0029]-[0073] | 1, 7-9 |
| Y | CN 106096077 A (XINXIANG MEDICAL UNIVERSITY) 09 November 2016 (2016-11-09) description abstract, description paragraphs [0029]-[0073] | 2-6, 10-20 |
| Y | CN 106503856 A (CHINA UNIVERSITY OF PETROLEUM (EAST CHINA)) 15 March 2017 (2017-03-15) description abstract, description paragraphs [0038]-[0080] | 2-6, 10-20 |
| A | CN 108664719 A (XINXIANG MEDICAL UNIVERSITY) 16 October 2018 (2018-10-16) entire document | 1-20 |

☐ Further documents are listed in the continuation of Box C.    ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **19 January 2022** | **11 February 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2021/131105**

| Patent document cited in search report | | | Publication date (day/month/year) | Patent family member(s) | Publication date (day/month/year) |
|---|---|---|---|---|---|
| CN | 113450868 | A | 28 September 2021 | None | |
| CN | 113450882 | A | 28 September 2021 | None | |
| CN | 106096077 | A | 09 November 2016 | None | |
| CN | 106503856 | A | 15 March 2017 | None | |
| CN | 108664719 | A | 16 October 2018 | None | |

Form PCT/ISA/210 (patent family annex) (January 2015)

**REFERENCES CITED IN THE DESCRIPTION**

**Patent documents cited in the description**

- CN 2020113431805
- CN 2020110330817